# FASCICULE DE BREVET EUROPEEN

(11) **EP 1 145 683 B1**
(45) Date de publication et mention de la délivrance du brevet: **24.11.2004**
(21) Numéro de dépôt: 00401879.2
(22) Date de dépôt: 30.06.2000
(51) Int. Cl.: A61B 10/00

(54) **Ustensile pour le prélèvement de la protubérance annulaire d'un bovin**
Instrument zur Probeentnahme von der Protuberancia annularis
Instrument for bovine annular protuberance sampling

(43) Date de publication de la demande: 17.10.2001
(73) Titulaire: Sarl Artbois, 76520 Boos (FR)
(72) Inventeur: Legeai, Thierry, 76520 Boos (FR); Venturini, Maurice, 76200 Dieppe (FR)
(74) Mandataire: Kohn, Philippe

(56) Documents cités:
- DE-C- 19 745 847
- FR-A- 2 588 748
- US-A- 4 800 896
- US-A- 4 984 367
- US-A- 5 084 005
- US-A- 5 348 023

## Description

La présente invention concerne un ustensile pour effectuer un prélèvement d'un échantillon de viscère aux fins d'analyses médicales ou vétérinaires.

L'invention concerne notamment un ustensile pour effectuer un prélèvement de la protubérance annulaire située dans la cavité occipitale d'un bovin.

Afin d'assurer une politique sanitaire efficace, il s'est avéré nécessaire de pouvoir réaliser des campagnes massives de prélèvement d'échantillons de viscères cervicaux des bovins afin d'effectuer des analyses vétérinaires pour déterminer si l'animal sur lequel on a effectué un prélèvement est atteint d'une maladie telle que la maladie dégénérative dite "de la vache folle".

Dans le cas du bovin, le viscère concerné est constitué par la protubérance annulaire située dans le crâne du bovin.

Pour accéder à cette protubérance annulaire qui est adjacente à une partie de la moelle épinière, il est nécessaire d'accéder à la partie arrière de la base du crâne du bovin, puis de dégager l'orifice occipital contenant la protubérance annulaire.

Dans cette situation, il faut pouvoir effectuer le prélèvement de l'échantillon constitué par la protubérance annulaire avec une efficacité maximale compte tenu du nombre de prélèvement à effectuer, tout en évitant d'endommager l'échantillon avant les opérations d'analyse, ainsi qu'en respectant des mesures d'hygiène et de propreté.

Traditionnellement, l'opération de prélèvement est effectuée au moyen d'un scalpel ou d'un outil tranchant similaire permettant de découper la protubérance annulaire, et plus précisément de la détacher de la cavité, en forme générale de tuyau, dans laquelle elle est logée.

En effet, la protubérance annulaire est rattachée aux parois de la cavité occipitale par différents organes tels que par exemple des terminaisons nerveuses et elle est aussi reliée par une de ses extrémités à la partie adjacente de la moelle épinière.

L'opération consistant à détacher la protubérance annulaire est donc une opération complexe à réaliser au moyen d'un scalpel et elle nécessite une grande habileté de la part de celui ou celle qui effectue le prélèvement.

Une fois la découpe effectuée, il est alors nécessaire de recueillir l'échantillon dans les conditions mentionnées précédemment.

L'opération de prélèvement de l'échantillon nécessite donc globalement, selon l'état de la technique, de faire appel à au moins deux outils, l'un de découpe et l'autre de prélèvement, pour recueillir l'échantillon et elle est particulièrement complexe à mettre en oeuvre.

S'agissant de chaque outil de découpe et de prélèvement, il est de plus nécessaire de les nettoyer entre chaque opération de prélèvement afin de ne pas polluer un échantillon par les restes de l'échantillon prélevé précédemment qui sont déposés sur les outils.

FR-A-2 588 748 décrit un ustensile utilisé pour la thrombectomie selon le préambule de la revendication 1, avec un profil rectangulaire.

Compte tenu de ce qui précède, il s'est avéré souhaitable de pouvoir disposer d'un nouvel outillage pour réaliser de tels prélèvements qui remédie aux inconvénients qui viennent d'être mentionnés et qui soit de préférence d'un coût réduit de manière à pouvoir être jetable, c'est-à-dire de pouvoir disposer d'un outillage distinct pour chaque prélèvement.

Dans ce but, l'invention propose un ustensile pour effectuer un prélèvement d'un échantillon de viscère, notamment de la protubérance annulaire située dans la cavité occipitale d'un bovin, aux fins d'analyses médicales ou vétérinaires, selon la revendication 1.

Grâce à la conception selon l'invention, on dispose ainsi d'un ustensile ou outil unique qui permet d'effectuer de manière ergonomique et aisée l'opération de prélèvement de l'échantillon.

Sa conception particulièrement simple permet de le réaliser de manière très économique, par exemple en une seule pièce moulée en matière plastique, et il peut ainsi être à usage unique, c'est-à-dire que l'ustensile peut être produit à un coût très faible et jeté après l'opération de prélèvement, ou bien constituer un moyen temporaire de transfert de l'échantillon compte tenu dans la cuillère de prélèvement.

Selon d'autres caractéristiques de l'invention :
- les tronçons longitudinaux du bord de la cuillère sont reliés au tronçon transversal d'extrémité libre par des tronçons de liaison réalisés sous la forme de tronçons tranchants ;
- chaque tronçon tranchant est réalisé à la faveur d'une réduction de l'épaisseur de la cuillère au voisinage de son bord périphérique de manière à réaliser un fil coupant ;
- la face concave de la cuillère comporte au moins une protubérance destinée à retenir l'échantillon dans la cuillère lors de son prélèvement ;
- chaque protubérance comporte une face avant inclinée pour faciliter l'introduction de l'ustensile d'arrière en avant, et une face transversale arrière de retenue qui s'étend dans un plan perpendiculaire à l'axe longitudinal de la cuillère.

D'autres caractéristiques et avantages de l'invention apparaîtront à la lecture de la description détaillée qui va suivre pour la compréhension de laquelle, on se reportera aux dessins annexés parmi lesquels :
- la figure 1 est une vue en perspective de l'ustensile selon l'invention ;

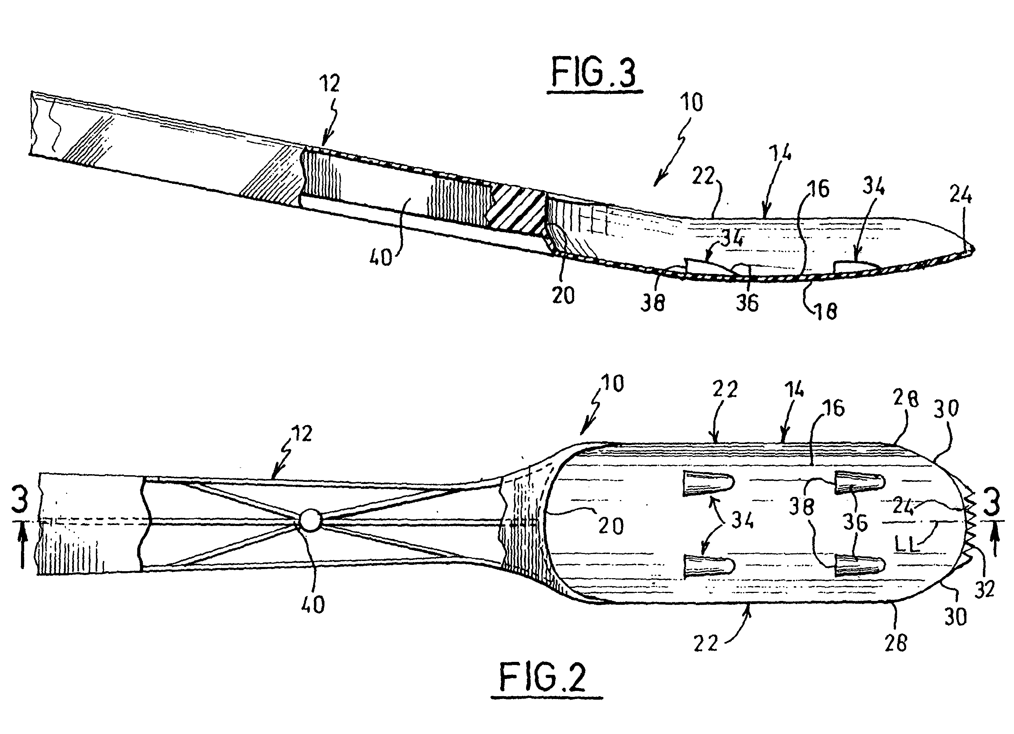

- la figure 2 est une vue de dessus de l'ustensile de la figure 1 ;
- la figure 3 est une vue en section longitudinale selon la ligne 3-3 de la figure 1.

Dans le mode de réalisation représenté aux figures, l'ustensile présente une symétrie générale de conception par rapport à un plan longitudinal vertical médian passant par son axe longitudinal L-L.

Afin de faciliter la compréhension de la description et des revendications, on utilisera une orientation verticale de la cuillère, c'est-à-dire que celle-ci s'étend dans un plan général horizontal avec sa face concave orientée verticalement vers le haut tandis que sa face convexe sensiblement parallèle est orientée verticalement vers le bas.

L'ustensile 10 est principalement constitué d'un manche 12 de manipulation de forme générale allongée selon un axe longitudinal central L-L.

On a représenté aux figures un ustensile 10 réalisé conformément aux enseignements de l'invention qui est plus particulièrement ici adaptée au prélèvement de la protubérance annulaire située dans la cavité occipitale d'un bovin.

Le manche 12 se prolonge au-delà de son extrémité avant par une cuillère 14 de prélèvement.

La cuillère est de forme générale allongée selon l'axe L-L et de profil en arc de cylindre en étant délimitée verticalement vers le haut par une face concave 16 et verticalement vers le bas par une face convexe 18.

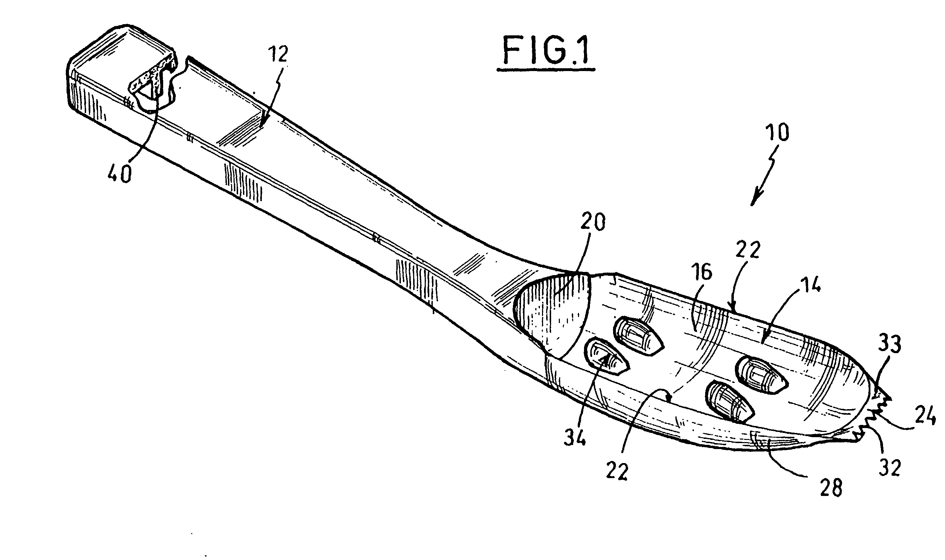

La cuillère 14 est délimitée longitudinalement vers l'arrière par une face transversale arrière 20 d'orientation générale verticale et de profil légèrement arrondi comme on peut le voir notamment à la figure 1.

Le profil concave 16 de la cuillère est, en section par un plan vertical transversal perpendiculaire à l'axe L-L, un profil en arc de cercle qui est sensiblement constant tout le long de la cuillère de manière à conférer à celle-ci un profil globalement en arc de cylindre.

La cuillère est délimitée latéralement par deux bords longitudinaux parallèles et opposés 22 et elle est délimitée longitudinalement vers l'avant par un bord transversal avant d'extrémité libre 24 dont les extrémités transversales opposées sont reliées aux extrémités longitudinales avant 28 des bords longitudinaux 22 par deux tronçons de liaison 30.

Les bords longitudinaux 22 sont des bords rectilignes parallèles tandis que le bord transversal avant 24 est lui aussi sensiblement rectiligne et perpendiculaire à l'axe L-L tandis que les tronçons de liaison 30 peuvent être rectilignes ou légèrement courbes pour assurer le raccordement entre les bords 22 et 24.

Conformément à une caractéristique essentielle de l'invention, les différents tronçons longitudinaux 22 et transversales 24 du bord périphérique général de la cuillère 14 sont réalisés sous la forme de tronçons tranchants permettant de réaliser des opérations de découpe en manipulant l'ustensile 10.

Chaque bord ou tronçon tranchant est de préférence réalisé à la faveur d'un amincissement de matière, c'est-à-dire grâce à une épaisseur réduite de la cuillère au voisinage de son bord périphérique par rapport à l'épaisseur moyenne de la cuillère entre ses faces concave 16 et convexe 18.

Le bord transversal d'extrémité libre avant 24 est un tronçon tranchant et il peut comporter aussi des dents 32 qui s'étendent longitudinalement vers l'avant pour constituer un bord tranchant et denté à la manière d'un couteau à dents, aussi appelé couteau-scie.

Afin de réaliser plus aisément un fil tranchant, notamment s'agissant du tronçon d'extrémité libre avant 24, il peut être souhaitable de former un léger méplat 33 dont l'intersection avec l'enveloppe externe de la face inférieure convexe 18 définie le fil tranchant.

Dans le mode de réalisation représenté aux figures, la face interne concave 16 de la cuillère de prélèvement 14 comporte quatre protubérances identiques 34 de retenue de l'échantillon prélevée.

Les quatre protubérances sont ici agencées sous la forme de deux paires de protubérances alignées longitudinalement l'une derrière l'autre.

Chaque protubérance formée en relief ou en saillie verticalement vers le haut dans la face concave 16 présente une face avant 36 globalement inclinée par rapport au plan du fond de la cuillère et une face transversale arrière de retenue 38 qui est d'orientation verticale et perpendiculaire à l'axe L-L.

De préférence, et notamment pour faciliter la fabrication de la cuillère par une opération de moulage en matière plastique en une seule pièce, la face avant 36 de chaque protubérance 34 est en forme de bossage arrondi et incliné.

La conception selon l'invention dans laquelle la cuillère 14 se situe sensiblement dans le prolongement longitudinal du manche 12 en formant éventuellement un léger angle d'environ 10° par rapport à ce dernier permet, de réaliser aisément l'ustensile 10 par moulage en matière plastique en une seule opération, l'opération de moulage permettant aussi de réaliser un manche 12 qui est creux avec des nervures de renfort 40.

Dans le cas d'un ustensile pour le prélèvement de la protubérance annulaire d'un bovin, la largeur transversale de la cuillère, c'est-à-dire la distance séparant ses deux bords ou tronçons longitudinaux 22 est d'environ 30 millimètres, tandis que la longueur de la cuillère est d'environ 80 millimètres.

Dans le cas du prélèvement mentionné précédemment sur un bovin, l'ustensile qui vient d'être décrit s'utilise de la manière suivante.

Après avoir dégagé l'accès à la cavité occipitale, l'utilisateur, qui est par exemple un vétérinaire, introduit longitudinalement, d'arrière en avant, la cuillère en tenant celle-ci de préférence à l'envers, c'est-à-dire avec sa concavité tournée vers le bas.

Après avoir introduit la cuillère le long d'une des parois de la cavité en forme de tuyau, l'utilisateur peut ensuite effectuer une rotation dans l'un ou l'autre sens de manière à découper la protubérance annulaire grâce aux bords tranchants longitudinaux 22.

De même, lors de l'introduction de la cuillère, son bord transversal avant tranchant 24, 32 participe à l'opération de découpe.

Les différents bords tranchants permettent de séparer l'échantillon de la moelle épinière adjacente et de découper les nerfs ou terminaisons nerveuses.

Une fois ces opérations de découpe et de séparation terminées, l'utilisateur peut introduire à nouveau longitudinalement la cuillère, cette fois-ci à l'endroit, pour recueillir la protubérance annulaire et l'extraire hors de la cavité.

Lors de l'introduction, les protubérances ou bossages en saillie 34 ne gênent pas l'introduction car leur face avant 36 est inclinée, tandis qu'ils participent à la retenue de l'échantillon pour permettre sont extraction d'avant en arrière, grâce à leur face transversale arrière verticale 38.

L'ustensile selon l'invention tel qu'il vient d'être décrit permet donc d'effectuer des opérations de prélèvement de viscère sans avoir à recourir à un quelconque outil coupant ou tranchant supplémentaire et il facilite ensuite la manipulation de l'échantillon dans la mesure où il peut être réalisé à bas coût sous la forme d'un ustensile jetable.

L'invention n'est, bien entendu, pas limitée au prélèvement de la protubérance annulaire d'un bovin, mais elle peut par exemple aussi servir, en adaptant les dimensions du produit, à un prélèvement de moelle osseuse à l'intérieur d'un os long dans le canal médullaire de celui-ci.

## Revendications

1. Ustensile (10) pour effectuer un prélèvement d'un échantillon de viscère, notamment de la protubérance annulaire située dans la cavité occipitale d'un bovin, aux fins d'analyses médicales ou vétérinaires, du type comportant un manche (12) de manipulation à l'extrémité avant duquel est agencée une partie creuse formant cuillère de prélèvement (14) dont le bord périphérique comporte un tronçon transversal (26) d'extrémité libre sensiblement perpendiculaire à l'axe général (L-L) de l'ustensile (10) et réalisé sous la forme d'un tronçon tranchant et comporte deux tronçons longitudinaux (22) parallèles audit axe et réalisés sous la forme de tronçons tranchants, pour réaliser la découpe de l'échantillon préalablement à son extraction, **caractérisé en ce que** le tronçon transversal d'extrémité libre tranchant (26) comporte des dents de scie (30), **en ce que**, en section transversale par un plan perpendiculaire à l'axe longitudinal de la cuillère (14), la face concave (16) de cette dernière présente un profil en arc de cercle, et **en ce que** l'ustensile est réalisé en une seule pièce par moulage en matière plastique.

2. Ustensile selon la revendication précédente, **caractérisé en ce que** la face concave (16) de la cuillère comporte au moins une protubérance (34) destinée à retenir l'échantillon dans la cuillère (14) lors de son prélèvement.

3. Ustensile selon la revendication précédente, **caractérisé en ce que** chaque protubérance (34) comporte une face avant inclinée (36) pour faciliter l'introduction de l'ustensile d'arrière en avant, et une face transversale arrière (38) de retenue qui s'étend dans un plan perpendiculaire à l'axe longitudinal (L-L) de la cuillère (14).

4. Ustensile selon l'une quelconque des revendications précédentes, **caractérisé en ce que** les tronçons longitudinaux (22) du bord de la cuillère (14) sont reliés au tronçon transversal d'extrémité libre (26) par des tronçons de liaison (30) courbes réalisés sous la forme de tronçons tranchants.

5. Ustensile selon l'une quelconque des revendications précédentes, **caractérisé en ce que** chaque tronçon tranchant (22, 26, 30) est réalisé à la faveur d'une réduction de l'épaisseur de la cuillère (140) au voisinage de son bord périphérique de manière à réaliser un fil coupant.

## Claims

1. Utensil (10) for extracting a sample of viscera, particularly from the brain stem located in the cranial cavity of cattle, for medical or veterinary analysis purposes, of the type comprising a manipulation handle (12), at the front end of which is arranged a hollow part forming an extraction spoon (14), and the peripheral edge of which comprises a transverse free end portion (26) roughly perpendicular to the overall axis (L-L) of the utensil (10) and produced in the form of a cutting portion and comprises two longitudinal portions (22) parallel to said axis and produced in the form of cutting portions, for cutting the sample before extracting it, **characterized in that** the transverse cutting free end portion (26) has sawteeth (30), **in that**, in cross section in a plane perpendicular to the longitudinal axis of the spoon (14), the concave face (16) thereof has a profile in the shape of an arc of a circle, and **in that** the utensil is made as a single piece by moulding of plastic.

2. Utensil according to the preceding claim, **characterized in that** the concave side (16) of the spoon comprises at least one projection (34) intended to hold the sample in the spoon (14) while it is being extracted.

3. Utensil according to the preceding claim, **characterized in that** each projection (34) has an inclined front face (36) to make it easier for the utensil to be introduced forwards, and a transverse retaining rear face (38) which runs in a plane perpendicular to the longitudinal axis (L-L) of the spoon (14).

4. Utensil according to any one of the preceding claims, **characterized in that** the longitudinal portions (22) of the edge of the spoon (14) are connected to the transverse free end portion (26) by connecting portions (30) produced in the form of cutting portions.

5. Utensil according to any one of the preceding claims, **characterized in that** each cutting portion (22, 26, 30) is produced by virtue of a reduction in the thickness of the spoon (140) near its peripheral edge so as to produce a cutting filament.

## Patentansprüche

1. Vorrichtung (10) zum Entnehmen einer Eingeweide-Probe, insbesondere aus der Varoli, die sich im Okzipitalraum eines Rindes befindet, um medizinische oder veterinärische Analysen vorzunehmen, wobei die Vorrichtung von dem Typ ist, der einen Handhabungsgriff (12) aufweist, an dessen vorderem Ende sich ein hohler Abschnitt befindet, der den Entnahmelöffel (14) bildet, dessen Umfangsrand ein transversales Teilstück (26) am freien Ende, das zu der allgemeinen Achse (L-L) der Vorrichtung (10) im Wesentlichen senkrecht ist und in Form eines Schneideteilstücks verwirklicht ist, und zwei longitudinale Teilstücke (22), die zu der Achse parallel sind und in Form schneidender Teilstücke verwirklicht sind, umfasst, um das Abschneiden der Probe vor seiner Extraktion zu verwirklichen, **dadurch gekennzeichnet, dass** das schneidende transversale Teilstück (26) am freien Ende Sägezähne (30) aufweist, dass die konkave Fläche (16) des Löffels (14) in einem Querschnitt längs einer Ebene senkrecht zur Längsachse des Löffels (14) ein kreisbogenförmiges Profil hat und dass die Vorrichtung durch Formung aus Kunststoff verwirklicht ist.

2. Vorrichtung nach dem vorhergehenden Anspruch, **dadurch gekennzeichnet, dass** die konkave Fläche (16) des Löffels wenigstens einen Vorsprung (34) aufweist, der dazu bestimmt ist, die Probe in dem Löffel (14) zu halten, wenn sie entnommen wird.

3. Vorrichtung nach dem vorhergehenden Anspruch, **dadurch gekennzeichnet, dass** jeder Vorsprung (34) eine geneigte vordere Fläche (36), um das Einführen der Vorrichtung von hinten nach vom zu erleichtern, und eine hintere transversale Haltefläche (38), die sich in einer Ebene senkrecht zur Längsachse (L-L) des Löffels (14) erstreckt, aufweist.

4. Vorrichtung nach einem der vorhergehenden Ansprüche, **dadurch gekennzeichnet, dass** die longitudinalen Teilstücke (22) des Randes des Löffels (14) mit dem transversalen Teilstück (36) des freien Endes durch gekrümmte Verbindungsteilstücke (30) verbunden sind, die in Form schneidender Teilstücke verwirklicht sind.

5. Vorrichtung nach einem der vorhergehenden Ansprüche, **dadurch gekennzeichnet, dass** jedes schneidende Teilstück (22, 26, 30) durch eine Verringerung der Dicke des Löffels (140) in der Nähe seines Umfangsrandes verwirklicht ist, um einen Schneidedraht zu verwirklichen.
